(19) Europäisches Patentamt
European Patent Office
Office européen des brevets

(11) Veröffentlichungsnummer: **0 011 758**
**B1**

(12) # EUROPÄISCHE PATENTSCHRIFT

(45) Veröffentlichungstag der Patentschrift: **23.09.81**

(51) Int. Cl.³: **C 07 C 175/00,** C 07 D 319/06

(21) Anmeldenummer: **79104439.9**

(22) Anmeldetag: **12.11.79**

(54) **Verfahren zur Herstellung von Polyenaldehyden.**

(30) Priorität: **25.11.78 DE 2851051**

(43) Veröffentlichungstag der Anmeldung: **11.06.80 Patentblatt 80/12**

(45) Bekanntmachung des Hinweises auf die Patenterteilung: **23.09.81 Patentblatt 81/38**

(84) Benannte Vertragsstaaten: **BE CH DE FR GB NL**

(56) Entgegenhaltungen:
**FR-A-1 495 335**
**H. MAYER et al. »Carotenoids« 1971, BIRKHAUSER, Basel, Stuttgart, Seiten 445 bis 448**

(73) Patentinhaber: **BASF Aktiengesellschaft, Carl-Bosch-Strasse 38, D-6700 Ludwigshafen (DE)**

(72) Erfinder: **Jaedicke, Hagen, Dr. Dipl.-Chem., Budapester Strasse 49, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Verfahren zur Herstellung von Polyenaldehyden

Die vorliegende Erfindung betrifft ein allgemein anwendbares, neues Verfahren zur Herstellung von Polyenaldehyden der allgemeinen Formel I

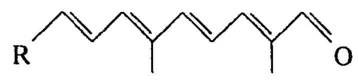

(I)

in der R einen organischen Rest bedeutet.

Speziell betrifft die Erfindung die Herstellung des wichtigen Lebensmittelfarbstoffes β-Apo-8'-carotinal Ia

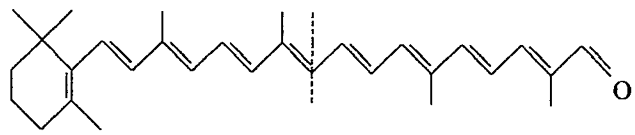

(I a)

also die Herstellung eines Polyenylaldehyds, in welchem R für den $C_{19}$-Rest der linken Formelseite steht.

Polyenverbindungen werden allgemein nach der Wittig'schen Ylid-Synthese durch Umsetzung des Ylids eines Phosphoniumsalzes oder eines Phosphonates mit einer Carbonylverbindung hergestellt. Demgemäß wurde auch das β-Apo-8'-carotinal aus verschiedenen Bausteinen zusammengefügt (vgl. Otto Isler »Carotenoids«; Birkhäuser Verlag Basel und Stuttgart 1971, Seiten 445—448). Alle diese Synthesen haben die gemeinsame Schwierigkeit, daß es sich um eine Reaktion handelt, bei welcher zwei Aldehydfunktionen zugegen sind, nämlich diejenige, die mit dem Ylid reagieren soll, und diejenige, welche für das Apocarotinal erhalten bleiben soll. Die letztgenannte Aldehydfunktion mußte daher durch Acetalisierung geschützt werden, wie es aus folgender Umsetzung (s. loc. cit) bekannt ist:

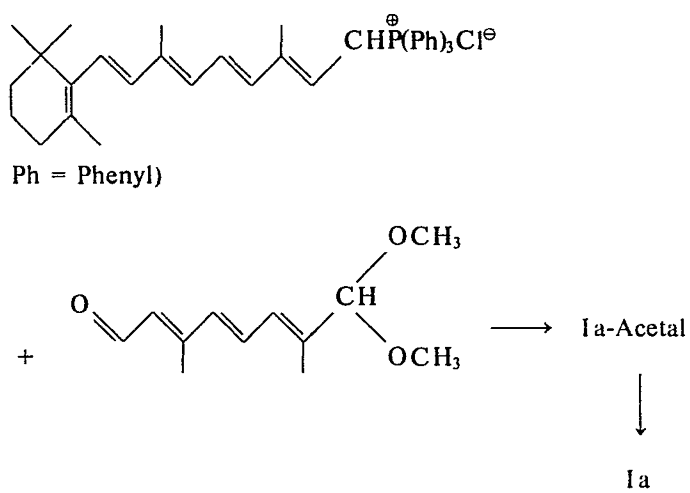


Daß diese Verfahrensweise umständlich ist, bedarf keiner näheren Erläuterung, zumal das Problem der spezifischen Reaktion einer Aldehydfunktion hier nur auf das Problem der spezifischen einseitigen Acetalisierung verschoben ist.

Aus der FR-PS 1 495 335 ist weiterhin ein Verfahren zur Herstellung von Polyenaldehyden nach der Wittig'schen Ylid-Synthese durch Umsetzen des Ylids einer Phosphoniumverbindung mit einem Dialdehyd bekannt. Gemäß diesem Verfahren werden jedoch ausschließlich symmetrische Dialdehyde umgesetzt.

Der Erfindung lag daher die Aufgabe zugrunde, die Polyenaldehyde I, darunter insbesondere das β-Apo-8'-carotinal Ia auf einfachere Weise herzustellen als bisher.

Es wurde nun überraschenderweise gefunden, daß man Polyenaldehyde der allgemeinen Formel I

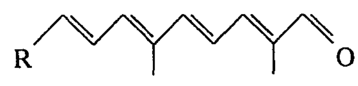

(I)

in der R einen organischen Rest bedeutet, in sehr großer Selektivität erhält, wenn man

2,6-Dimethyl-octa-2,4,6-trien-1,8-dial II

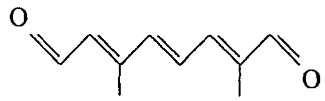

(II)

mit äquimolaren oder annähernd äquimolaren Mengen eines Ylids der allgemeinen Formel III

$$R'—\overset{\ominus}{C}H—\overset{\oplus}{P}R'_3 \qquad \text{(III)}$$

in der die Reste R' für gleiche oder verschiedene organische Reste stehen, oder einer Verbindung der allgemeinen Formel IV

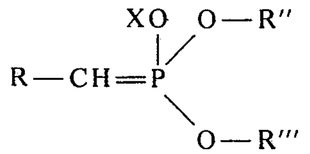

(IV)

in der R'' und R''' für gleiche oder verschiedene organische Reste und X für ein Kation steht, umsetzt.

Dieses Verfahren ist bemerkenswert, weil es jeglicher Erwartung widersprach, daß bei dieser Synthese unter den angegebenen stöchiometrischen Bedingungen ausschließlich die 8-al-Funktion des Dialdehyds II reagiert. Diese Spezifität der Umsetzung ist von außerordentlicher Bedeutung, da bei der Reaktion der 1-al-Funktion des Dialdehyds II mit III oder IV ein Isomeres von I gebildet würde, das praktisch nicht von dem gewünschten Produkt abtrennbar ist. Für die Herstellung des β-Apo-8'-carotinals Ia wäre die erfindungsgemäße Umsetzung schon nicht mehr akzeptabel, wenn mehr als etwa 4% der 1-al-Gruppen anstelle der 8-al-Gruppen reagieren würden, da das Verkaufslimit für die Lebensmittelqualität des Lebensmittelfarbstoffs β-Apo-8'-carotinal 96% der natürlichen Form darstellt. Es versteht sich von selbst, daß es prinzipiell von Vorteil ist, II und III in etwa äquimolaren Mengen einzusetzen. Prinzipiell ist dies jedoch nicht unbedingt erforderlich, da — wie oben schon beschrieben — praktisch keine 1-Ylidierung stattfindet, solange die 8-Ylidierung noch nicht abgeschlossen ist. Liegt also II im Überschuß über III oder IV vor, so erhält man ausschließlich (bezogen auf III oder IV) die Verbindung I von der sich das überschüssige II in aller Regel abtrennen läßt. Auch ein geringer Überschuß des Ylids des Phosphoniumsalzes oder Phosphonats kann aus verfahrenstechnischen Gründen vorteilhaft sein, da in diesem Falle zunächst eine vollständige Umsetzung zu I stattfindet, wonach I eventuell zum beiderseitig ylidierten Dialdehyd II weiterreagiert. Dieses meist unerwünschte Folgeprodukt läßt sich bei nur geringem Anteil aus dem kristallinen Gemisch in der Regel gut abtrennen.

Besondere Bedeutung hat das erfindungsgemäße Verfahren im Hinblick auf die Herstellung von Ia. Dies ist nicht nur wegen der spezifischen einseitigen Ylidreaktion der Fall, sondern gilt auch, weil die hierbei als Ausgangsverbindung benötigte Ylid IIIa

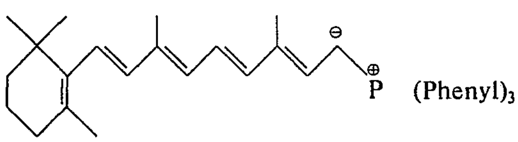

(III a)

von der Synthese des Vitamin A (Axerophtol) in großtechnischen Mengen durch Nutzbarmachung der Vitamin-A-Endlaugen zur Verfügung steht. Hierzu müssen diese Laugen, die entweder Vitamin A oder dessen Acetat enthalten, lediglich mit einer Säure HX und einem tertiären Phosphin $PR_3'$ in bekannter Weise in das Phosphoniumsalz überführt werden, welches durch Umsetzen mit einem Protonenakzeptor in das Ylid IIIa überführt werden kann. Die Gewinnung der Axerophthylphosphonate bzw. deren Ylide aus den Vitamin-A-Endlaugen zwecks Herstellung von β-Apo-8'-carotinal sind weniger vorteilhaft.

Der als Ausgangsverbindung benötigte Dialdehyd II ist eine Verbindung, die zwar in der oben genannten Monographie »Carotenoids« auf Seite 410 erwähnt, aber bisher unseres Wissens noch nicht näher charakterisiert wurde. Er ist nach an sich bekannten Methoden erhältlich. Beispielsweise erhält man ihn durch Wittig-Reaktion eines 3-Methyl-fumardialdehyd-1-monoacetals mit einem 4,4-Dimethoxy-3'-methyl-but-2-en-1-yl-triphenylphosphoniumsalz und anschließende Verseifung.

Die Phosphorylide der allgemeinen Formel III können in an sich bekannter Weise, z. B. durch Einwirken starker Basen auf die Ihnen zugrundeliegenden Phosphoniumsalze hergestellt werden. Näheres hierüber ist u. a. der zusammenfassenden Arbeit von Tripett (Quart. Reviews, Bd. 17 (1963) S. 406 ff.) zu entnehmen.

Für die erfindungsgemäße Umsetzung ist es zweckmäßig, daß man das Phosphorylid gleich in dem für die Wittig-Synthese vorgesehenen Lösungsmittel und gegebenenfalls sogar in Gegenwart des Dialdehyds II aus dem entsprechenden Phosphoniumsalz herstellt. Die benötigten Phosphoniumsalze können in an sich bekannter und einfacher Weise durch Umsetzen der entsprechenden organischen Halogenide mit den Phosphinen $PR_3'$ oder aber durch Umsetzen der entsprechenden organischen Hydroxyverbindungen und einer Säure HX mit den Phosphinen $PR_3'$ hergestellt werden. Die tertiären Phosphine $PR_3'$ können, wie aus den umfangreichen Untersuchungen zur Ylid-Synthese allgemein bekannt ist, prinzipiell beliebig sein, weil die Art der Umsetzung von der unterschiedlichen Reaktionsfähigkeit der beiden Aldehydfunktionen in II bestimmt wird, nicht jedoch — oder nur in untergeordnetem Ausmaß — von der Reaktionsfähigkeit der Phosphoniumsalze. Da bis jetzt jedoch das Triphenylphosphin am billigsten ist und auch keine besonderen Aufarbeitungs- und Umweltprobleme bedingt, wird dieses in großtechnischen Mengen verfügbare Phosphin bevorzugt.

Als Säure HX verwendet man bevorzugt starke Säuren wie etwa Chlorwasserstoff, Bromwasserstoff oder Schwefelsäure, da die Phosphoniumsalze mit diesen Anionen, wie ebenfalls allgemein bekannt ist, gegen Selbstzersetzung stabiler sind, als mit Anionen schwacher Säuren.

Als Ylide der allgemeinen Formel III verwendet man bevorzugt solche, in denen R' für gleiche oder verschiedene Arylreste oder Cyclohexyl stehen.

Als starke Basen zur Herstellung der Phosphorylide können die für Wittig-Synthesen üblichen Basen verwendet werden. Werden die Phosphorylide gleich in dem für die Wittig-Reaktion benötigten Reaktionsmedium hergestellt, so sind Alkali- und Erdalkalihydroxide, Alkalihydride, Alkaliamide und Alkali- und Erdalkalialkoholate zu empfehlen.

Werden die Phosphorylide jedoch vor der eigentlichen Reaktion isoliert, so können außer den genannten Basen auch Basen wie Phenyl-Lithium oder Butyl-Lithium verwendet werden.

Auch Äthylenoxid (s. Angew. Chem. 80 (1968, Seite 535 ff.) und überschüssiges Phosphorylid können unter bestimmten Bedingungen die Rolle der starken Base übernehmen.

Als Lösungsmittel für die Herstellung der Phosphorylide sowie für die Durchführung der Wittig-Reaktion kommen die für Wittig-Synthesen üblichen Lösungsmittel, beispielsweise aliphatische oder aromatische Kohlenwasserstoffe, wie Hexan, Octan, Cyclohexan, Benzol, Toluol und Xylol und deren Halogenierungsprodukte, ferner Alkohole, wie Methanol, Äthanol, Isopropanol, Butanole, Hexanole, Cyclohexanol und Cyclooctanol, auch Glykole, sowie Äther, wie Diisopropyläther, Äthylenglykoldimethyläther, Tetrahydrofuran, Dimethyltetrahydrofuran und Dioxan oder Gemische aus ihnen in Frage. Besonders geeignet sind polare organische Lösungsmittel, wie Methanol, Äthanol, Formamid, Dimethylformamid, N-Methylpyrrolidon, Hexamethylphosphorsäuretriamid, Acetonitril und Dimethylsulfoxid oder Gemische dieser Lösungsmittel. Auch in Wasser oder in Wasser enthaltenden Gemischen läßt sich das Verfahren der Erfindung ausführen.

Anstelle der Ylide der allgemeinen Formel III können — wie allgemein für Wittig-Reaktionen bekannt ist — auch Verbindungen der Formel IV

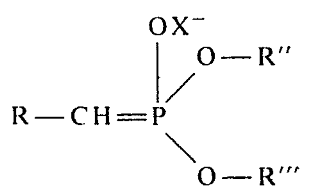

(IV)

in der R'' und R''' Alkyl-, Cycloalkyl-, Aralkyl- oder Arylreste bedeuten, eingesetzt werden. Man erhält sie in an sich bekannter Weise, z. B. durch Umsetzen der entsprechenden Halogenide oder Tosylate mit Triestern der phosphorigen Säure und anschließendes Umsetzen der erhaltenen Phosphonsäureester der Formel

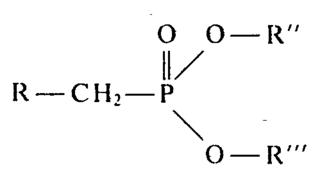

mit starken Basen als Protonenakzeptoren. Als starke Basen und als Lösungsmittel können hierbei die gleichen verwendet werden, wie sie für die Ylidherstellung beschrieben sind.

Zur Durchführung der erfindungsgemäßen Umsetzung von 2,6-Dimethyl-octa-2,4,6-trien-1,8-dial II mit den Yliden III oder den Verbindungen IV legt man zweckmäßig das Dial II in einem Lösungsmittel vor, fügt etwa stöchiometrische Mengen einer starken Base hinzu und versetzt die erhaltene Suspension bei der Reaktionstemperatur langsam mit der Lösung eines Ylids oder einer Verbindung IV und läßt das Reaktionsgemisch noch etwa 1 bis 2 Stunden nachreagieren. Man kann jedoch auch eine

Lösung des Dials II und des Ylids vorlegen und hierzu portionsweise die starke Base addieren oder aber zunächst aus den Phosphoniumsalzen oder Phosphonsäureestern mit starken Basen III bzw. IV herstellen und zu dieser Lösung langsam das Dial II addieren. Die Reaktionstemperatur kann etwa −70 bis 100° C, vorzugsweise 0 bis +70° C betragen.

Da die Wittig-Reaktion an sich bekannt ist, erübrigen sich weitere Ausführungen.

Die organischen Reste R in III oder IV können grundsätzlich beliebig sein, so daß das erfindungsgemäße Verfahren einen neuen vorteilhaften Weg zu prinzipiell allen Aldehyden der Formel I eröffnet. Für die Herstellung von Carotinoiden, carotinoidähnlichen Verbindungen sowie von deren Vor- und Zwischenprodukten sind solche Verbindungen I von besonderem Interesse, in denen R ein gesättigter oder ungesättigter $C_1$- bis $C_{40}$-Kohlenwasserstoffrest ist. Vor allem kommen Reste mit dem Kohlenstoffgerüst des Vitamin A oder Bruchstücken davon in Betracht, wobei die Kohlenstoffkette und der endständige 6-Ring Methyl- oder Äthylgruppen als Substituenten tragen kann und wobei die Reste durchlaufend konjungiert, ungesättigt oder partiell oder gänzlich abgesättigt sein können. Auch derartige Reste mit 1 bis 2 äthylenisch ungesättigten Bindungen oder solche mit einer oder mehreren Äthergruppierungen, vor allem im 6-Ring, sind für weitere Synthesen von Interesse.

Die Mannigfaltigkeit der präparativen Möglichkeiten wird schließlich noch dadurch erweitert, daß die Aldehyde I ihrerseits Ylidreaktionen mit Phosphoniumsalzen oder Phosphonaten eingehen können. Alle diese Synthesen, durch welche das Strukturelement

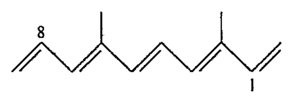

in Carotinoide eingeführt werden soll, werden durch das erfindungsgemäße Verfahren erheblich vereinfacht, indem man ohne Hilfsoperationen zunächst eine 8-Verknüpfung und ggf. dann noch eine 1-Verknüpfung vornimmt.

## Beispiel 1

### Herstellung von β-Apo-8′-carotinal (Ia)

Eine Suspension aus 2,6 g (15,83 mmol) 2,6-Dimethyl-2,4,6-octatrien-1,8-dial, 1,85 g pulverisiertem Magnesiumhydroxid und 50 ml Toluol wurde zum Sieden erhitzt und im Laufe von 60 Minuten mit einer Lösung aus 10,0 g (15,90 mmol) Axerophtyl-triphenylphosphoniumhydrogensulfat (IIIa) in 30 ml Methanol versetzt, noch weitere 30 Minuten erhitzt und nach dem Abkühlen mit verdünnter Essigsäure angesäuert. Anschließend wurde das Reaktionsgemisch mit 20 ml Wasser versetzt und die wäßrige Phase abgetrennt. Die toluolische Lösung wurde noch 2mal bei 60° C mit je 50 ml 60%igem wäßrigem Dimethylformamid und mit 30 ml Wasser gewaschen. Das Toluol wurde dann unter vermindertem Druck bis auf ein Restvolumen von 12 ml abdestilliert und dieses mit 54 ml Methanol und 3,2 ml einer 30vol.-%igen wäßrigen Schwefelsäure versetzt. Nach 24stündigem Rühren bei Raumtemperatur konnten 4,55 g (69% der Theorie) reines β-Apo-8′-carotinal in kristalliner Form abgetrennt werden, die nach HPLC-Analyse (Cyclohexan/Ethylacetat 5 : 1, Li Chrosorb Si 60) zu mehr als 96% aus dem gewünschten all-trans Produkt (identisch mit authentischem Muster) bestehen. NMR-spektroskopisch konnte weder im Kristallisat, noch in der Mutterlauge das regioisomere Produkt (welches durch Reaktion der Aldehydfunktion in 8-Stellung von 2,6-Dimethyl-octa-2,4,6-trien-1,8-dial entstehen würde) — z. B. anhand des charakteristischen Dubletts für das Aldehydproton dieser Verbindung — nachgewiesen werden.

## Beispiel 2

### Herstellung von 2,6,11-Trimethyl-11-(4,4-dimethyl-2,6-dioxan-1-yl)-dodecapenta-2,4,6,8,10-en-1-al

Eine Suspension aus 1 g (6,09 mmol) II, 15 ml Toluol und 0,93 g pulverisiertem Calciumhydroxid wurde in der Siedehitze im Laufe von 30 Minuten mit einer Lösung von 2,9 g (6,21 mmol) 3-(4,4-Dimethyl-2,6-dioxan-1-yl)-but-2-en-1-yl-triphenylphosphoniumchlorid

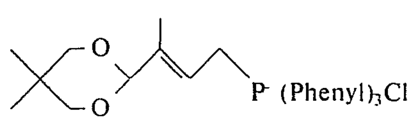

und 8 ml Methanol versetzt und danach noch weitere 2 Stunden unter Rückflußkühlung erhitzt. Nach

dem Abkühlen wurde das Gemisch mit wäßriger Essigsäure angesäuert, wonach die organische Phase analog Beispiel 1 aufgearbeitet wurde. Hierbei fiel der oben genannte Aldehyd, der die Struktur

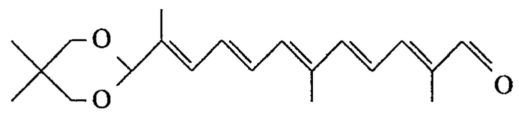

hat, beim Einengen der toluolischen Lösung als kristalliner Rückstand in 95% Ausbeute an. Der erhaltene Aldehyd ist neu.

$^1$H-NMR (Lösungsmittel $CDCl_3$; TMS als innerer Standard; $\delta$ in ppm):
9,46 (s, 1H); 6,2–7,2 (m, 7H); 4,81 (s, 1H); 3,6 (4H); 1,9–2,1 (9H);
1,25 (s, 3H) und 0,78 (s, 3H).

Das NMR-Spektrum zeigte keinerlei Hinweis auf das Vorhandensein des anderen Regioisomeren, beispielsweise war kein Dublett für das Aldehydproton sichtbar.

**Patentansprüche**

1. Verfahren zur Herstellung von Polyenaldehyden der allgemeinen Formel I

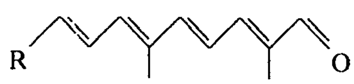

(I)

in der R einen organischen Rest bedeutet, dadurch gekennzeichnet, daß man 2,6-Dimethyl-octa-2,4,6-trien-1,8-dial II

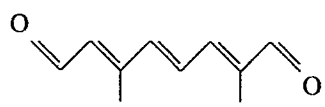

(II)

mit äquimolaren oder annähernd äquimolaren Mengen eines Ylids der allgemeinen Formel III

$$R—\overset{\ominus}{\bar{C}}H—\overset{\oplus}{P}R'_3 \qquad (III)$$

in der die Reste R' für gleiche oder verschiedene organische Reste stehen, oder einer Verbindung der allgmeinen Formel IV

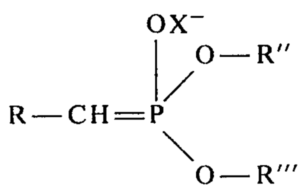

(IV)

in der R'' und R''' für gleiche oder verschiedene organische Reste und X für ein Kation steht, umsetzt.

2. Verfahren gemäß Anspruch 1, dadurch gekennzeichnet, daß man das Ylid eines Axerophtylphosphoniumsalzes mit II zum $\beta$-Apo-8'-carotinal umsetzt.

**Claims**

1. A process for the preparation of a polyene-aldehyde of the general formula I

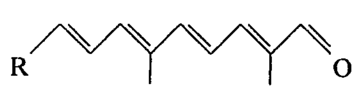

(I)

where R is an organic radial, characterized in that 2,6-dimethylocta-2,4,6-triene-1,8-dial II

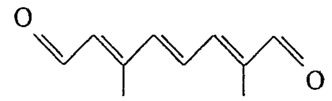

(II)

is reacted with an equimolar or about equimolar amount of an ylide of the general formula III

$$R—\overset{\ominus}{\bar{C}}H—\overset{\oplus}{P}R'_3 \qquad \text{(III)}$$

where the radicals R′ are identical or different organic radicals, or of a compound of the general formula IV

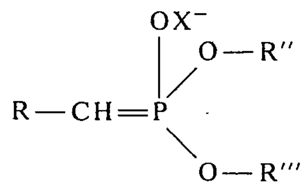

(IV)

where R″ and R‴ are identical or different organic radicals and $X^-$ is a cation.

2. A process as claimed in claim 1, characterized in that the ylide of an axerophthyl-phosphonium salt is reacted with compound II to give β-apo-8′-carotinal.

**Revendications**

1. Procédé pour la préparation d'aldéhydes polyéniques de formule générale I

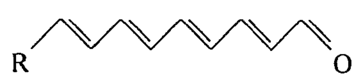

(I)

dans laquelle R représente un radical organique, caractérisé en ce qu'on fait réagir un 2,6-diméthyl-octa-2,4,6-triène-1,8-dial II

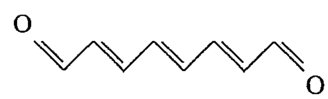

(II)

avec des quantités équimolaires ou approximativement équimolaires d'un ylure de formule générale III

$$R'—\overset{\ominus}{\bar{C}}H—\overset{\oplus}{P}R'_3 \qquad \text{(III)}$$

dans laquelle les radicaux R′ sont des radicaux organiques semblables ou différents, ou d'un composé de formule générale IV

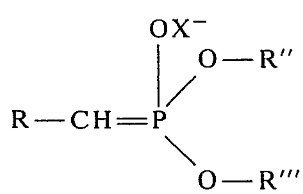

(IV)

dans laquelle R″ et R‴ sont mis pour des radicaux organiques semblables ou différents et $X^-$ pour un cation.

2. Procédé selon la revendication 1, caractérisé en ce qu'on fait réagir l'ylure d'un sel d'axérophtylphosphonium avec II pour obtenir le β-apo-8′-caroténal.